# FASCICULE DE BREVET EUROPEEN

(11) **EP 1 920 676 B1**
(45) Date de publication et mention de la délivrance du brevet: **02.05.2012**
(21) Numéro de dépôt: 07301292.4
(22) Date de dépôt: 02.08.2007
(51) Int. Cl.: A45D 34/00, A45D 40/00, A61M 35/00

(54) **Dispositif vibrant et procédé de maquillage utilisant un tel dispositif**
Schwingvorrichtung und diese Vorrichtung verwendendes Schminkverfahren
Vibrating device and method of applying make-up using such a device

(30) Priorité: 21.08.2006 FR 0653416
(43) Date de publication de la demande: 14.05.2008
(73) Titulaire: L'Oréal, 75008 Paris (FR)
(72) Inventeur: Gueret, Jean-Louis, 75016 PARIS (FR)
(74) Mandataire: Tanty, François

(56) Documents cités:
- WO-A-2004/047583
- US-A- 5 519 292
- US-A1- 2006 034 904

## Description

La présente invention concerne un procédé de traitement cosmétique des matières kératiniques, notamment un procédé de maquillage. L'invention concerne également les ensembles pour la mise en oeuvre d'un tel procédé.

Il est connu de munir un doigt ou la main d'un dispositif comportant une source vibrante afin de réaliser un massage ou une stimulation.

Les brevets US 3 623 481 et 5 356 369 et les demandes WO 96/32915, US 2003/0181835, WO 02/051348 et US 2004/0020241 décrivent de tels dispositifs.

Le brevet US 7 050 360 décrit une montre incluant une source vibrante afin de réveiller plus efficacement l'utilisateur.

Le brevet US-A-2006-34904 décrit un ensemble comportant un produit cosmétique dans une capsule plate et un dispositif d'aide à l'application sur une partie plate de la peau avec une source d'ultrasons vibrante, elle aussi plate.

On connaît par la demande de brevet WO 2006/020577 une brosse à mascara configurée pour osciller à grande vitesse de manière à modifier la viscosité du mascara.

Cette brosse présente différents inconvénients.

L'utilisateur ne peut modifier la direction des vibrations de la brosse. Son usage est d'autre part limité à l'application de mascara. Enfin, sa réalisation est relativement complexe et son coût est élevé pour un usage limité à la quantité de produit initialement contenue dans le récipient associé.

Il existe un besoin pour perfectionner encore les ensembles pour l'application d'un produit sur les matières kératiniques et améliorer la qualité d'un maquillage ou les performances des produits.

Il existe encore un besoin pour proposer aux consommateurs de nouveaux procédés de traitement cosmétiques.

L'invention vise à répondre à l'un au moins de ces besoins.

### Ensembles

L'invention a pour objet, selon un premier de ses aspects, un ensemble d'application d'un produit cosmétique ou dermatologique comme défini dans la revendication 1 comportant :
- un produit de maquillage et/ou un applicateur configuré pour appliquer le produit sur des matières kératiniques,
- un dispositif d'aide à l'application du produit, ce dispositif comportant des moyens de fixation amovible à au moins un doigt et une source vibrante permettant de produire des vibrations.

L'invention offre un moyen simple et efficace pour améliorer le maquillage et/ou l'application d'un produit de soin et/ou améliorer les performances du produit.

Les vibrations peuvent faciliter le transfert de produit sur la surface d'application, l'étalement du produit sur la surface d'application, le chargement en produit de l'applicateur ou du doigt, l'homogénéité de l'application, le glissement du doigt ou de l'applicateur sur la zone traitée et peut permettre de masser celle-ci.

L'invention peut permettre d'améliorer l'application réalisée au moyen des applicateurs connus grâce à la production des vibrations.

La couvrance obtenue et les effets optiques éventuels liés à la quantité de produit déposée peuvent être mieux contrôlés par l'utilisateur.

En fonction des rhéologies des produits utilisés, la couche déposée peut être mieux lissée. Avec un produit visqueux, la séparation des cils ou sourcils peut être meilleure. Lorsque le produit comporte des fibres, le glissement de ces dernières et leur orientation le long des cils peuvent être améliorés.

Dans le cas de l'application au moyen d'un pinceau, par exemple de vernis à ongles, la régularité de la couche déposée et l'épaisseur du produit peuvent être améliorées. Il est possible grâce à l'invention de diminuer la formation de stries inesthétiques lors de l'application du produit sur l'ongle.

L'applicateur peut être agencé pour retenir le produit par capillarité en l'absence de vibrations et les vibrations peuvent amener le produit à s'écouler vers la région à traiter ou vers un organe d'application.

Enfin, lorsque l'applicateur comporte un bloc de mousse ou une membrane floquée par exemple, il est possible grâce à l'invention de diminuer le risque d'enlever le produit déjà déposé sur la peau en frottant l'applicateur sur cette dernière. La couche de produit déposée peut être plus épaisse et mieux répartie quelque soit la nature de l'applicateur, même lorsque ce dernier est dépourvu d'un revêtement suffisamment doux, par exemple d'un flocage.

Lorsque l'applicateur comporte un dispositif de pulvérisation, il est possible grâce à l'invention d'améliorer la dispersion du produit dans l'air et de mieux maquiller toutes les surfaces, y compris des petites surfaces ou des surfaces accidentées.

L'ensemble peut comporter un moyen de conditionnement contenant le produit et/ou l'applicateur et le dispositif d'aide à l'application. Ce moyen de conditionnement est par exemple une boîte, un sachet, un coffret, un blister.

L'ensemble peut contenir un produit de maquillage ou de soin. L'ensemble peut comporter un applicateur et le produit à appliquer. L'applicateur peut être agencé pour fermer un récipient contenant le produit.

Les moyens de fixation peuvent être agencés pour permettre la fixation sur l'un au moins de l'index, du pouce, du majeur, sur la troisième phalange d'un doigt, la deuxième phalange d'un doigt, la première phalange d'un doigt.

Les moyens de fixation peuvent comporter au moins une portion annulaire ou semi-annulaire à travers laquelle le doigt peut être introduit.

Les moyens de fixation peuvent comporter au moins une pince pouvant enserrer le doigt. La pince est par exemple formée d'une bague interrompue. L'utilisation d'un moyen de fixation élastiquement déformable tel qu'une pince peut permettre un contact étroit entre le dispositif d'aide à l'application et le doigt ou l'applicateur sur lequel le dispositif est monté.

Les moyens de fixation peuvent comporter au moins une structure pouvant être refermée sur le doigt, notamment deux portions à assembler sur le doigt.

Les moyens de fixation peuvent encore comporter une portion en forme de doigt de gant, de gant ou de dé.

Les moyens de fixation peuvent comporter une bague réalisée d'une seule pièce par moulage avec au moins une portion du boitier du dispositif d'aide à l'application.

L'ensemble peut comporter un organe de commande du fonctionnement de la source vibrante. Cet organe de commande peut être à déclenchement par enfoncement. L'organe de commande peut être disposé de telle sorte que l'enfoncement s'effectue à la mise en place du doigt. En variante ou additionnellement, l'organe de commande peut être disposé de telle sorte que l'enfoncement s'effectue en réponse à la mise en contact du dispositif avec l'applicateur. L'organe de commande peut être à contact fugitif, le fonctionnement de la source vibrante cessant dès que l'organe de commande est relâché.

Le dispositif peut comporter, à proximité de l'organe de commande, au moins un relief antidérapant d'une matière élastomère, par exemple entourant l'organe de commande, afin de réduire le glissement de l'applicateur au contact de la source vibrante et/ou filtrer les vibrations transmises. L'applicateur peut appuyer sur le relief antidérapant tout en déclenchant le fonctionnement de la source vibrante. Le relief antidérapant est formé par exemple d'une ou plusieurs nervures, notamment des nervures annulaires.

L'ensemble peut comporter deux organes de commande du fonctionnement de la source vibrante, notamment sur deux faces respectives du dispositif.

L'ensemble peut comporter un moteur permettant de produire des vibrations, notamment un moteur entraînant une masselotte ou un excentrique, un vibreur piézoélectrique ou électromécanique. La source vibrante peut encore comporter un moteur entraînant une roue dentée et un frotteur constitué par exemple d'une lame élastique s'appliquant sur cette roue dentée. La vitesse de rotation du moteur peut être par exemple comprise entre 4500 et 12000 tr/mn, par exemple 7000 et 12000 tr/mn, par exemple 4500 et 800 tr/mn. La tension d'alimentation utilisée peut être de 1,5 V à 3 V par exemple. La fréquence fondamentale des vibrations produites peut être comprise entre 1 et 500 Hz, par exemple 10 à 300 Hz ou 50 à 200 Hz. La fréquence des fibrations est par exemple supérieure ou égale à 20 Hz. La fréquence peut varier en fonction de l'endroit ou de la pression avec laquelle les vibrations sont transmises. Les vibrations peuvent être produites de façon intermittente ou continue. Le moteur peut être contenu dans un carter métallique avec la masselotte excentrée, ce carter ayant par exemple une forme de disque. Le cas échéant, le carter peut servir à l'alimentation électrique du moteur.

Le moteur ou le carter ci-dessus peut être monté sur un amortisseur en élastomère, qui permet par exemple la transmission des vibrations à la fréquence fondamentale et filtre les vibrations harmoniques.

Le dispositif peut comporter une source électrique. L'utilisation d'une pile bouton peut s'avérer avantageuse pour diminuer l'encombrement du dispositif. En variante, le dispositif peut être dépourvu de source électrique, celle-ci étant présente dans l'applicateur. En cas d'utilisation d'une pile bouton et d'un moteur en forme de disque, la pile et le moteur peuvent être face à face, côte à côte ou la face de l'un peut être en regard de la tranche de l'autre.

L'ensemble peut comporter un organe de réglage permettant à l'utilisateur de régler la fréquence et/ou l'amplitude des vibrations, et/ou de régler l'orientation des vibrations par rapport aux moyens de fixation.

Les vibrations peuvent être orientées transversalement à l'axe longitudinal de l'applicateur ou parallèlement à celui-ci ou autrement encore.

L'amplitude des vibrations de l'applicateur lors de l'application peut par exemple être inférieure ou égale à 5 mm, mieux inférieure ou égale à 3 mm, des micro-vibrations de l'applicateur étant préférables à des vibrations de plus forte amplitude. L'amplitude des vibrations peut éventuellement être plus grande lors du prélèvement du produit dans un récipient ou lors de la traversée d'un organe d'essorage.

L'ensemble peut comporter au moins deux applicateurs différents et/ou deux produits différents.

Les moyens de fixation peuvent être agencés, le cas échéant, pour permettre la fixation du dispositif sur l'applicateur et/ou sur un récipient contenant le produit.

Le dispositif d'aide à l'application peut comporter une première articulation permettant de faire tourner la source vibrante autour d'un premier axe de rotation par rapport aux moyens de fixation et une deuxième articulation permettant de faire tourner la source vibrante autour d'un deuxième axe perpendiculaire au premier, relativement aux moyens de fixation.

### Applicateurs

Le dispositif d'aide à l'application peut être utilisé pour permettre l'application d'un produit au moyen d'un applicateur sur une région à maquiller. Il peut être utilisé au moment du prélèvement, de l'application, ou postérieurement à l'application.

Grâce à l'invention, le dispositif d'aide à l'application peut être associé à l'applicateur de manière souple et non contraignante, de telle sorte qu'il est aisé à l'utilisateur de décider de la direction des vibrations transmises à l'applicateur et de faire varier cette direction en fonction de l'application à effectuer et des effets de maquillage souhaités.

Le dispositif d'aide selon l'invention peut être utilisé avec tous types d'applicateurs.

L'applicateur peut par exemple être fixé au doigt, par exemple collé ou fixé par un adhésif ou par coopération de crochets et de boucles ou par tout autre moyen. En variante, l'applicateur peut être simplement maintenu par l'utilisateur entre ses doigts.

L'applicateur peut être agencé pour appliquer un produit sur les cils ou les cheveux, et comporter par exemple une brosse ou un peigne. La brosse peut comporter une âme torsadée et des poils pris entre les spires de l'âme ou être réalisée autrement encore. Le peigne peut être par exemple réalisé d'une seule pièce par moulage de matière plastique.

L'applicateur peut encore comporter un pinceau destiné à l'application sur les ongles, les lèvres ou la peau. L'applicateur peut comporter des poils collés ou agrafés, des poils torsadés, un non-tissé, un feutre, un flocage, une mousse.

L'applicateur peut encore comporter un élément d'application capillaire, configuré pour retenir du produit par capillarité, comportant par exemple une paroi tubulaire et un élément intérieur définissant avec la paroi tubulaire un espace pour retenir le produit par capillarité. Un tel applicateur peut être utile, par exemple, pour appliquer un produit sur les ongles.

En variante, l'applicateur peut comporter un filetage ou une succession de stries annulaires, l'élément d'application étant par exemple formé par un empilement de disques. Un tel applicateur peut être destiné à l'application du produit sur les fibres kératiniques.

Dans un exemple de réalisation, l'applicateur peut comporter un embout, notamment un embout floqué, éventuellement élastiquement déformable.

Dans un exemple de réalisation, l'applicateur peut comporter un organe élastiquement compressible et/ou poreux, telle que par exemple une mousse ou un élastomère, éventuellement floqué.

Dans un exemple de réalisation, l'applicateur peut comporter un feutre.

L'applicateur peut être magnétique.

Dans certains exemples de réalisation, l'applicateur peut être monté à l'extrémité d'une tige, laquelle peut être flexible, ce qui peut contribuer à augmenter l'amplitude des vibrations de l'applicateur et/ou améliorer le confort à l'application.

L'applicateur peut comporter un organe de préhension. La forme de l'organe de préhension peut être choisie de manière à être adaptée à la forme d'un doigt, de manière à améliorer la transmission des vibrations.

La forme de l'organe de préhension peut également être adaptée à la forme du dispositif d'aide à l'application lui-même, de manière à améliorer la transmission directe des vibrations entre le dispositif et l'applicateur.

L'applicateur peut comporter ou non une réserve de produit. Dans ce cas, l'applicateur peut comporter un élément d'application alimenté en produit par une réserve de produit. Cette réserve peut être fixée de manière amovible ou non sur l'applicateur. Lorsque la réserve est présente à demeure sur l'applicateur pour alimenter l'élément d'application en produit, la paroi de la réserve peut par exemple servir à la préhension de l'applicateur.

L'applicateur peut comporter une réserve de produit communiquant avec un élément d'application ou un orifice de distribution par un conduit. En l'absence de vibrations, l'écoulement du produit dans le conduit peut être faible voire nul. En présence de vibrations, l'écoulement peut s'opérer. L'utilisateur, en amenant ou non son doigt ou la source vibrante au contact de l'applicateur ou en faisant vibrer ou non celle-ci, peut agir sur l'écoulement de produit.

Lorsque l'applicateur ne comporte pas de réserve de produit, le produit peut être par exemple contenu dans un récipient, et l'applicateur être chargé en produit en étant introduit au moins partiellement dans ce récipient. Le récipient peut comporter ou non un organe d'essorage. L'applicateur peut comporter éventuellement un organe de fermeture d'un récipient contenant le produit à appliquer.

L'applicateur peut encore comporter un dispositif de pulvérisation d'un produit sur le visage ou le corps.

L'applicateur peut permettre de prélever la composition à appliquer dans un récipient pourvu d'un organe d'essorage ou sur un pain de produit.

Le contact entre le dispositif d'aide à l'application et l'applicateur peut être ponctuel ou étendu, selon par exemple l'amplitude, la fréquence et l'orientation des vibrations que l'on recherche, étant réglé par l'utilisateur lui-même selon la disposition et l'orientation du dispositif d'aide par rapport à l'applicateur.

L'applicateur peut éventuellement lui-même comporter une source vibrante, par exemple dans un organe de préhension de l'applicateur ou dans un capuchon de fermeture d'un récipient.

Le dispositif d'aide peut être dépourvu de tout moyen de fixation sur l'applicateur.

### Procédés de traitement cosmétique, notamment de maquillage

L'invention a encore pour objet un procédé de traitement cosmétique, dans lequel on applique un produit cosmétique sur des matières kératiniques après avoir fixé sur le doigt un dispositif d'aide à l'application comportant une source vibrante.

Le procédé peut permettre d'appliquer le produit au moyen du doigt en se servant de l'extrémité du doigt comme surface d'application.

En variante, le produit peut être appliqué au moyen d'un applicateur.

Le dispositif d'aide à l'application peut être sans contact avec l'applicateur lors de l'application du produit.

On peut amener le dispositif d'aide à l'application au contact de l'applicateur pour lui transmettre des vibrations lors de l'application. Les vibrations peuvent être exercées durant l'application du produit sur les matières kératiniques. En variante ou additionnellement, les vibrations peuvent être exercées durant le prélèvement du produit.

Les vibrations peuvent être produites pendant le prélèvement du produit et/ou pendant l'application du produit, de manière continue ou de manière discontinue ou intermittente.

Les matières kératiniques peuvent être constituées par la peau, les lèvres, des fibres kératiniques, notamment les cils, sourcils ou cheveux, ou encore les ongles, par exemple les ongles des mains ou des pieds.

Ce procédé peut être mis en oeuvre pour maquiller les fibres kératiniques, notamment les cils ou sourcils, ou une mèche de cheveu, et peut permettre d'obtenir une meilleure séparation des cils, et/ou dans le cas de l'utilisation d'un produit comportant des fibres, une meilleure orientation de celles-ci, et/ou une facilité de prélèvement.

Le procédé peut encore permettre d'obtenir un dépôt de produit plus lisse sur les cils ou sourcils ou la mèche de cheveu, et davantage de brillance.

Le procédé selon l'invention peut encore être mis en oeuvre pour appliquer un produit sur les ongles des mains ou des pieds, la peau ou les muqueuses, par exemple les lèvres, et peut alors permettre de déposer une couche plus épaisse et/ou plus lisse de produit. La brillance et la couvrance peuvent s'en trouver augmentées. La présence des vibrations peut encore permettre d'augmenter l'épaisseur de produit déposé. Les vibrations peuvent accroître l'accrochage du produit sur la surface traitée, par exemple dans le cas d'un rouge à lèvres ou d'un vernis à ongles.

Par ailleurs, dans le cas de l'application sur les ongles, l'invention peut permettre d'utiliser pour l'application du produit des poils plus gros et/ou plus rigides sans pour autant conduire à la formation de stries sur l'ongle. L'invention peut ainsi permettre, par exemple, de réduire le nombre de poils des pinceaux utilisés pour l'application du vernis à ongles.

Les vibrations transmises à l'applicateur peuvent aussi faciliter l'arrivée de produit vers l'applicateur, notamment lorsque l'applicateur comporte une réserve de produit alimentant une surface d'application.

L'invention peut également permettre d'étaler plus facilement le produit sur la surface à traiter.

Dans le cas d'un blush par exemple, l'invention peut permettre d'obtenir une meilleure uniformité de couleur, et dans le cas d'une ombre à paupières ou d'un produit anti-cernes, un dépôt plus homogène.

Le produit peut être appliqué à chaud ou à froid.

Le dispositif vibrant peut être utilisé pour la finition d'un maquillage, ou sur une région déjà maquillée ou chargée de produit au préalable, au moyen d'un applicateur sans utilisation du dispositif d'aide.

Le produit peut être prélevé dans un récipient en immergeant l'applicateur ou un doigt dans celui-ci. Lors du prélèvement, l'applicateur ou le doigt peuvent être soumis aux vibrations du dispositif vibrant, ce qui peut permettre d'obtenir le cas échéant une charge plus homogène de produit sur l'applicateur ou sur le doigt.

Lorsque le récipient comporte un organe d'essorage au travers duquel l'applicateur est retiré, l'applicateur peut également être soumis à des vibrations au moment du passage à travers l'organe d'essorage, ce qui peut permettre d'obtenir un essorage de l'applicateur différent de celui qui existe en l'absence de vibrations de l'applicateur. L'utilisateur peut ainsi par exemple choisir entre au moins deux degrés d'essorage de l'applicateur, selon que l'applicateur vibre ou non au moment de la traversée de l'organe d'essorage. L'organe d'essorage pourra présenter une ouverture nettement plus large que la tige éventuelle de l'applicateur.

Il est également possible, en variante, de faire vibrer au moyen du dispositif vibrant de l'invention le récipient contenant le produit et l'organe d'essorage, par exemple.

Indépendamment de la nature de l'applicateur et de celle du produit à appliquer, le procédé selon l'invention peut comporter le réglage par l'utilisateur d'une fréquence de vibration et/ou le réglage d'une amplitude de vibration, par exemple en agissant sur un organe de réglage.

Le procédé de maquillage peut comporter l'application d'un produit sur certaines parties du corps ou du visage en faisant vibrer l'applicateur ou la portion du corps humain et sur d'autres parties du corps ou du visage sans faire vibrer l'applicateur ou la portion du corps humain, afin d'obtenir des effets de maquillage différents, par exemple des brillances différentes.

Le procédé peut encore comporter le chargement de l'applicateur ou du doigt en produit, lorsque celui-ci se présente sous forme de poudre compactée, en faisant vibrer l'applicateur ou le doigt au contact de la poudre.

Le dispositif d'aide peut être utilisé pour faire vibrer un applicateur par l'intermédiaire du doigt ou directement. Un même dispositif peut être utilisé dans ces deux configurations, selon sa disposition sur l'utilisateur.

Le dispositif peut par exemple être disposé d'un côté ou de l'autre d'un doigt, entrant ainsi ou non en contact directement avec l'applicateur.

Le contact du dispositif avec l'applicateur peut s'effectuer par une face de l'applicateur orientée sensiblement perpendiculairement à un axe longitudinal de l'applicateur.

En variante, le contact entre l'applicateur et le dispositif d'aide peut s'effectuer par une face de l'applicateur orienté sensiblement parallèlement à un axe longitudinal de l'applicateur.

L'invention a encore pour objet, selon un autre de ses aspects, un procédé d'application d'un produit cosmétique de maquillage sur les matières kératiniques en vue de former un dépôt visible sur les matières kératiniques, comportant les étapes suivantes :
- former un dépôt de produit sur les matières kératiniques,
- simultanément à la formation du dépôt ou postérieurement au dépôt, soumettre ce dernier à un mouvement vibratoire au moyen d'un dispositif d'aide tel que défini ci-dessus,
- laisser le dépôt sur les matières kératiniques, le dépôt pouvant sécher.

Le produit peut par exemple être déposé sur les fibres kératiniques, notamment les cils, les sourcils ou cheveux, les muqueuses, les lèvres, les ongles des mains ou des pieds, les paupières, le contour des yeux, le visage et/ou le corps. Le procédé apparaît notamment très avantageux pour l'application d'un produit sur les ongles, les lèvres ou les fibres kératiniques.

Le produit est par exemple un produit destiné à être appliqué sur les ongles, la peau, les fibres kératiniques, notamment les cils, sourcils ou cheveux, ou les muqueuses, par exemple les lèvres, étant par exemple un mascara, un vernis à ongles, un rouge à lèvres, un brillant à lèvres, un fond de teint, un blush, une ombre à paupières, un produit pour le contour des yeux (*concealer*)*,* un eye-liner, un anti-cernes, un autobronzant, cette liste n'étant pas limitative.

Le produit peut notamment être différent d'un dentifrice et d'un produit destiné à la dermo-abrasion et peut notamment comporter des pigments, notamment non alimentaires. Le produit peut comporter des fibres, des paillettes ou d'autres éléments macroscopiques. Le produit peut présenter des propriétés magnétiques, le cas échéant.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente, de manière schématique, en élévation, un exemple d'ensemble selon l'invention,
- la figure 2 est une coupe longitudinale, schématique et partielle, du dispositif d'aide à l'application de l'ensemble de la figure 1,
- la figure 3 illustre l'utilisation de l'ensemble lors du prélèvement du produit,
- les figures 4 à 7 illustrent des variantes d'ensemble, lors de l'utilisation,
- la figure 8 représente en coupe longitudinale, schématique, une variante de réalisation du dispositif d'aide à l'application,
- les figures 9 et 10 représentent des exemples de systèmes de conditionnement,
- la figure 11 représente partiellement des exemples d'applicateur,
- la figure 12 représente en coupe longitudinale partielle un autre exemple d'applicateur,
- les figures 13 à 16 sont des vues d'autres exemples de dispositifs de conditionnement et d'application pouvant être utilisés avec le dispositif d'aide à l'application,
- les figures 17 à 19 représentent des exemples de positionnement sur le dispositif d'aide à l'application de l'organe de commande du fonctionnement de la source vibrante,
- les figures 20 à 24 illustrent d'autres exemples de moyens de fixation sur le doigt ou la main,
- les figures 25 et 26 illustrent deux manières parmi d'autres d'utiliser le dispositif d'aide à l'application,
- la figure 27 illustre le montage du dispositif d'aide à l'application sur un applicateur,
- la figure 28 illustre la possibilité de fixer le dispositif d'aide à l'application sur un récipient contenant le produit à appliquer,
- les figures 29 à 32 illustrent différentes possibilités d'orientations des vibrations relativement aux moyens de fixation,
- la figure 33 représente, en élévation, un dispositif d'aide dans lequel l'orientation de la source vibrante peut être modifiée par rapport aux moyens de fixation,
- la figure 34 représente un dispositif d'aide à l'application offrant une possibilité de réglage de l'amplitude des vibrations,
- la figure 35 représente en vue de dessus un autre exemple de dispositif réalisé conformément à l'invention,
- la figure 36 est une vue analogue à la figure 35 après une rotation d'un quart de tour du dispositif relativement aux moyens de fixation sur le doigt,
- la figure 37 est une vue de côté selon XXXVII de la figure 35,
- la figure 38 est une vue analogue à la figure 37 après rotation sur lui-même du dispositif,
- les figures 39 à 41 représentent en élévation différents exemples de positionnement d'un dispositif réalisé conformément à l'invention sur un applicateur,
- les figures 42 et 43 illustrent des détails de réalisation de variantes de dispositifs d'aide à l'application, et
- la figure 44 représente en élévation, avec arrachement, une variante de dispositif d'aide à l'application.

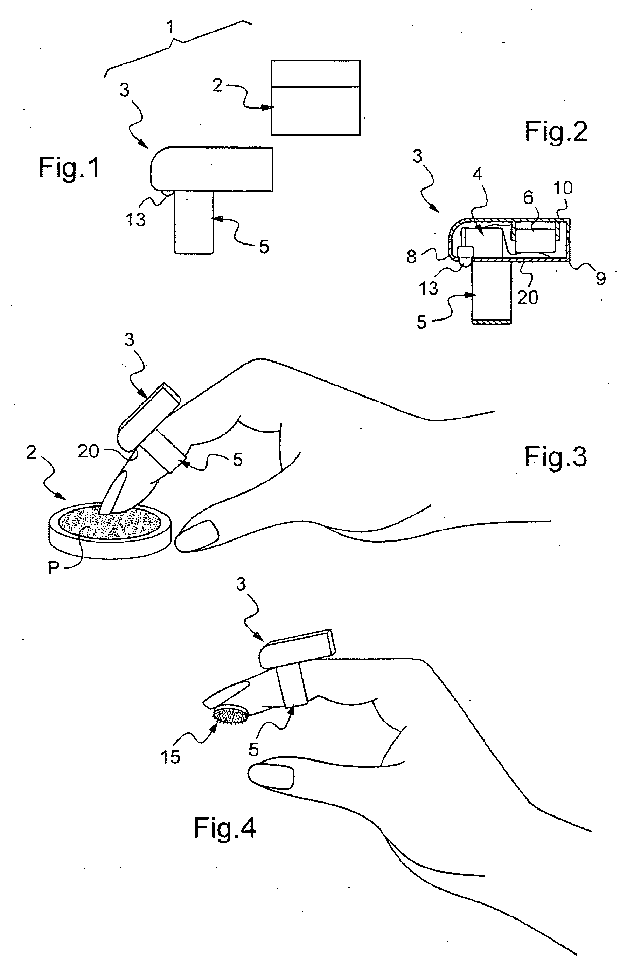

On a représenté à la figure 1 un ensemble 1 selon l'invention, encore appelé kit, comportant d'une part un récipient 2 contenant un produit à appliquer sur les matières kératiniques, par exemple un produit cosmétique ou dermatologique notamment un produit de maquillage, et d'autre part, un dispositif 3 d'aide à l'application.

Ce dispositif 3 comporte comme illustré à la figure 2 une source vibrante 4 et des moyens de fixation 5 sur une portion du corps, en l'espèce un doigt dans l'exemple illustré. Ces moyens de fixation 5 sont par exemple formés d'une bague, fermée ou fendue.

La source vibrante 4 comporte un ou plusieurs composants générant des vibrations lorsqu'alimentés en électricité et permet de transmettre ces vibrations à une surface d'application servant au prélèvement et/ou à l'application du produit, de façon à faciliter ce prélèvement et/ou améliorer les caractéristiques de l'application et/ou celles du produit.

Dans l'exemple considéré, le dispositif 3 comporte un boîtier 8 qui loge la source vibrante 4 et une source électrique 6 permettant d'alimenter en électricité la source vibrante 4, par exemple une ou plusieurs piles ou accumulateurs. Le dispositif 3 est relativement petit et le boîtier 8 est plat, formé par exemple par deux disques.

L'invention n'est pas limitée à un dispositif 3 dans lequel la source vibrante 4 est alimentée par une source électrique 6 logée dans le boîtier du dispositif, comme cela sera exemplifié plus loin.

La source vibrante 4 peut être réalisée de diverses manières, et comporte par exemple un moteur électrique permettant d'entraîner en rotation une masselotte ou un excentrique, la source vibrante étant par exemple choisie parmi celles utilisées pour réaliser les vibreurs des téléphones portables. La source vibrante 4 peut encore comporter un vibreur piézoélectrique ou électromécanique. Le moteur ci-dessus est par exemple un moteur en forme de disque. Le moteur peut être contenu avec la masselotte excentrée dans un carter, par exemple métallique, en forme de disque.

L'invention n'est pas limitée à une orientation particulière de l'axe de rotation du moteur et la source vibrante 4 peut être disposée de manière à générer des vibrations qui peuvent être orientées de diverses manières, comme cela sera détaillé plus loin.

Le boîtier 8 est par exemple réalisé avec au moins deux parties 9 et 10 qui sont assemblées après mise en place de la source vibrante 4 et de la source électrique 6 et des différents moyens de connexion électrique et de commande du fonctionnement.

Le caractère amovible ou non de la fixation des parties 9 et 10 l'une sur l'autre peut être choisi selon que l'on souhaite ou non permettre un remplacement de la source électrique 6 une fois que celle-ci est épuisée.

La source vibrante 4 peut être fixée par exemple à l'une des parties 9 et 10 et la source électrique 6 à l'autre partie, comme illustré, mais en variante, la source vibrante 4 et la source électrique 6 peuvent être fixées à la même partie 9 ou 10.

Les moyens de fixation 5 peuvent, le cas échéant, être réalisés d'une seule pièce par moulage de matière plastique avec l'une des parties, par exemple celle supportant la source électrique 6 et/ou la source vibrante 4.

Le dispositif 3 peut éventuellement être réalisé avec une trappe permettant de remplacer la source électrique 6 sans avoir à démonter le boîtier 8.

La mise en marche du dispositif 3 peut s'effectuer à l'aide d'un organe de commande 13 qui est par exemple à déclenchement par enfoncement et qui est par exemple agencé de manière à être actionné lors de la mise en place des moyens de fixation 5 sur le doigt, comme illustré à la figure 3.

L'organe de commande 13 peut être réalisé de diverses manières et peut comporter une lame métallique souple qui vient fermer un circuit électrique lorsque déformée par l'utilisateur.

L'organe de commande 13 peut être à contact fugitif, c'est-à-dire être agencé de telle sorte que le fonctionnement n'ait lieu que tant qu'une action est exercée dessus par l'utilisateur. En variante, le dispositif peut comporter une temporisation assurant un fonctionnement pendant une durée prédéfinie après un bref actionnement. En variante encore, le fonctionnement peut être commandé par une pression sur l'organe de commande 13 puis arrêté par une nouvelle pression.

Lorsque la source vibrante 4 est alimentée, le dispositif 3 transmet ses vibrations au doigt et à la surface d'application.

Dans l'exemple de la figure 3, le prélèvement du produit contenu dans le récipient 2 s'effectue avec le doigt et la source vibrante 4 peut fonctionner dès ce prélèvement, ce qui peut par exemple permettre d'améliorer celui-ci, notamment de faciliter le délitement du produit lorsque celui-ci est un pain ou une poudre compactée. En variante, le dispositif 3 ne fonctionne pas lors du prélèvement mais seulement lors de l'application. En variante encore, le dispositif 3 fonctionne lors du prélèvement et lors de l'application.

Le dispositif 3 d'aide à l'application peut encore être utilisé avec un applicateur, lequel peut se présenter sous diverses formes et notamment être inclus avec le produit dans l'ensemble 1. En variante, ce dernier peut ne comporter que le dispositif d'aide et l'applicateur.

Dans l'exemple de la figure 4, l'applicateur est par exemple un applicateur 15 agencé pour se fixer également sur le doigt, par exemple au moyen d'un adhésif ou autrement. Des exemples de tels applicateurs sont divulgués notamment dans les demandes de brevet US 2004/0031723 et FR 2 432 287.

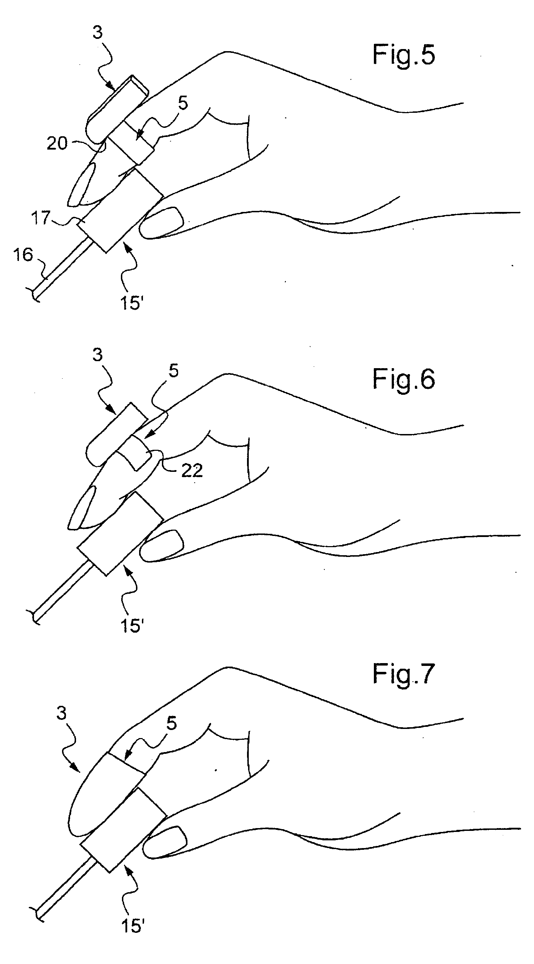

Dans l'exemple de la figure 5 notamment, l'applicateur 15' comporte une tige 16 et un organe de préhension 17 qui peut constituer également un organe de fermeture d'un récipient contenant le produit, l'organe de préhension 17 comportant par exemple à cet effet une jupe filetée intérieurement.

La tige 16 porte, du côté opposé à l'organe de préhension 17, un élément d'application qui peut être choisi en fonction du traitement à réaliser et qui n'a pas été représenté sur la figure 5.

L'organe de préhension 17 est par exemple saisi durant l'utilisation entre le pouce, l'index et le majeur et le dispositif 3 est par exemple porté par l'un de ces trois doigts, en l'espèce l'index dans l'exemple de la figure 5.

Dans les exemples des figures 3 à 5, les moyens de fixation 5 sont constitués par exemple par une bague dans laquelle le doigt est introduit, cette bague étant par exemple réalisée d'une seule pièce avec au moins une portion du boîtier 8.

Les moyens de fixation 5 peuvent encore être au moins partiellement rapportés sur le boîtier 8, notamment de façon amovible, afin de permettre l'utilisation de moyens de fixation ayant plusieurs tailles, en fonction de la grosseur des doigts de l'utilisateur.

Les moyens de fixation 5, notamment la bague illustrée, peuvent être encore réalisés le cas échéant dans un matériau élastiquement déformable, afin de faciliter l'adaptation à différentes grosseurs de doigts.

Lorsque le dispositif 3 est en place sur le doigt, il peut ne venir au contact de celui-ci que par l'intermédiaire des moyens de fixation 5, ou en variante, également par la face 20 du boîtier 8 tournée vers les moyens de fixation 5, ce qui peut améliorer éventuellement la transmission des vibrations au doigt. Les moyens de fixation peuvent le cas échéant comporter des moyens de réglage à la taille du doigt de l'utilisateur, par exemple grâce à une portion mobile relativement au boîtier 8.

Le dispositif 3 peut comporter éventuellement des moyens de réglage permettant d'agir sur l'étendue de la surface du dispositif 3 en contact avec le doigt, afin de transmettre plus ou moins facilement les vibrations.

Les moyens de fixation 5 peuvent occuper une position fixe relativement à l'axe longitudinal du boîtier 8. En variante, les moyens de fixation 5 peuvent être mobiles relativement au boîtier, afin par exemple de rapprocher ou d'éloigner la source vibrante 4 des moyens de fixation 5 et favoriser ou non le transfert des vibrations, en fonction par exemple du maquillage à réaliser.

Dans le cas où les moyens de fixation comportent une bague, celle-ci peut recouvrir une portion plus ou moins grande du doigt. La dimension axiale de la bague peut ainsi, par exemple, être sensiblement égale à la longueur du boîtier, voire supérieure à cette dernière.

L'invention n'est pas limitée à des moyens de fixation 5 particuliers et dans l'exemple de la figure 6, ceux-ci comportent une pince comportant deux branches arquées 22 venant enserrer entre elles le doigt. Cela peut accroître les possibilités d'utilisation du dispositif 3, notamment en permettant de le fixer sur la phalange souhaitée afin par exemple d'éloigner ou de rapprocher le dispositif 3 de la surface d'application. Cela peut également permettre une fixation plus facile sur des doigts de tailles différentes, voire directement sur une portion de l'applicateur ou du récipient.

Dans l'exemple de la figure 7, le dispositif 3 se présente sous la forme d'un doigt de gant ou d'un dé, selon la longueur du doigt qui est recouverte.

Les moyens de fixation 5 se présentent par exemple sous la forme d'un manchon ouvert à une extrémité, le dispositif 3 venant recouvrir entièrement l'extrémité distale du doigt, par exemple seulement la troisième phalange ou en variante les troisième et deuxième phalanges.

Le manchon précité peut être en un matériau rigide ou souple, selon par exemple le confort que l'on souhaite et la qualité de la transmission des vibrations recherchée.

Lorsque la transmission des vibrations doit se faire vers le doigt, une interface rigide entre le doigt et le dispositif peut être préférable afin de réduire l'amortissement des vibrations.

Lorsque la transmission des vibrations se fait par contact entre l'applicateur 15' et le dispositif 3, l'interface précitée peut être plus souple.

Dans l'exemple de la figure 7, l'organe de commande 13 est par exemple disposé à l'intérieur du dispositif 3 de telle sorte que l'introduction du doigt déclenche automatiquement le fonctionnement de la source vibrante. En variante, l'organe de commande 13 peut être actionné depuis l'extérieur du dispositif 3, par exemple au moment où ce dernier vient au contact de l'applicateur.

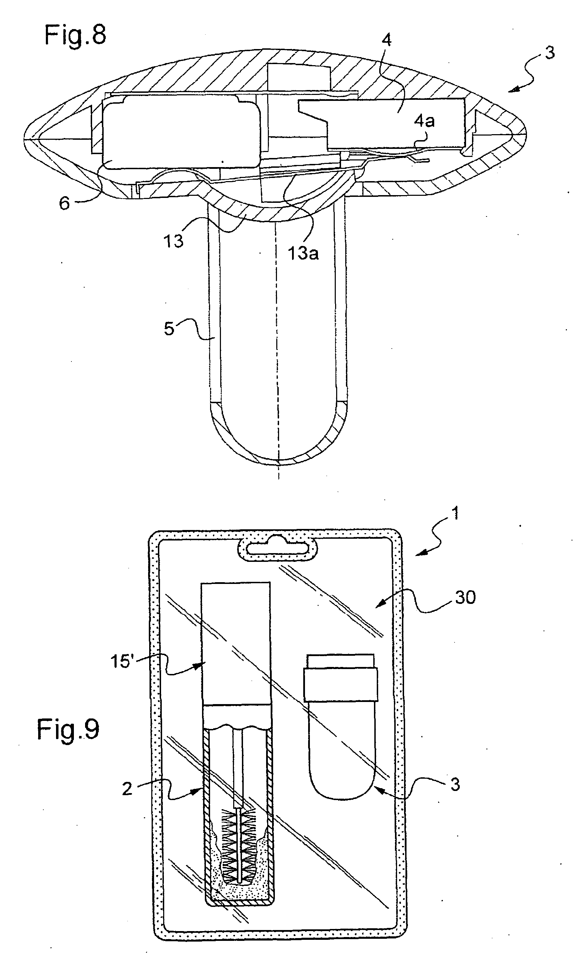

La figure 8 représente une variante de réalisation du dispositif d'aide à l'application 3.

On voit sur cette figure que l'organe de commande 13 peut être situé en regard des moyens de fixation 5.

L'organe de commande 13 peut comporter un poussoir en matière plastique servant à faire fléchir une lame conductrice 13a élastiquement déformable reliée à la source électrique 6 pour l'amener à toucher un contact 4a relié électriquement au moteur. La source vibrante 4 et la source électrique 6 sont disposées côte à côte, étant généralement aplaties parallèlement à l'axe longitudinal du dispositif 3.

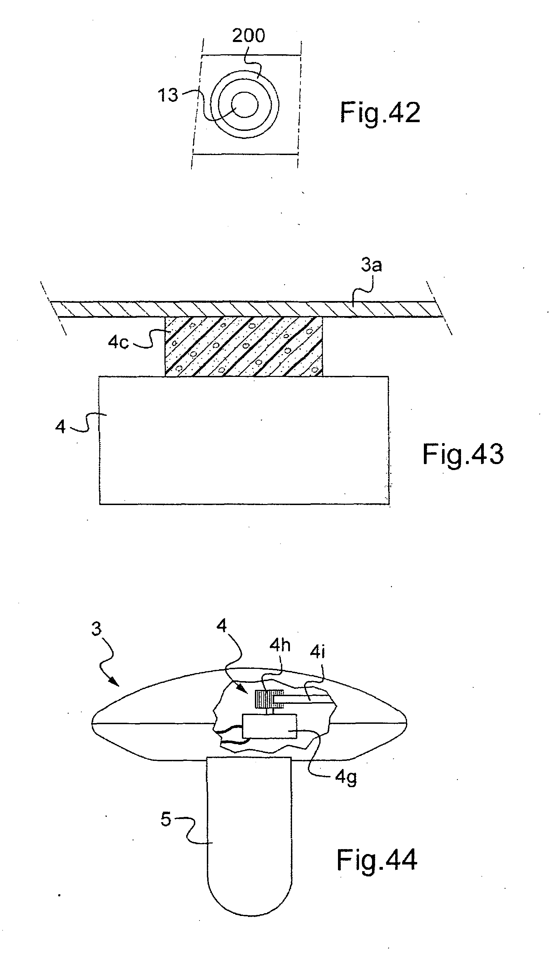

On a illustré à la figure 43 la possiblité pour la source vibrante 4 d'être reliée à une paroi 3a du dispositif 3 par l'intermédiaire d'un bloc élastiquement déformable 4c par lequel transitent les vibrations. Ce bloc 4c peut agir comme filtre de fréquences en atténuant les vibrations harmoniques tout en laissant passer les vibrations à la fréquence fondamentale.

On voit sur la figure 44 que la source vibrante 4 peut comporter un moteur 4g entrainant en rotation une roue dentée 4h et une lame 4i frottant sur la roue dentée 4h.

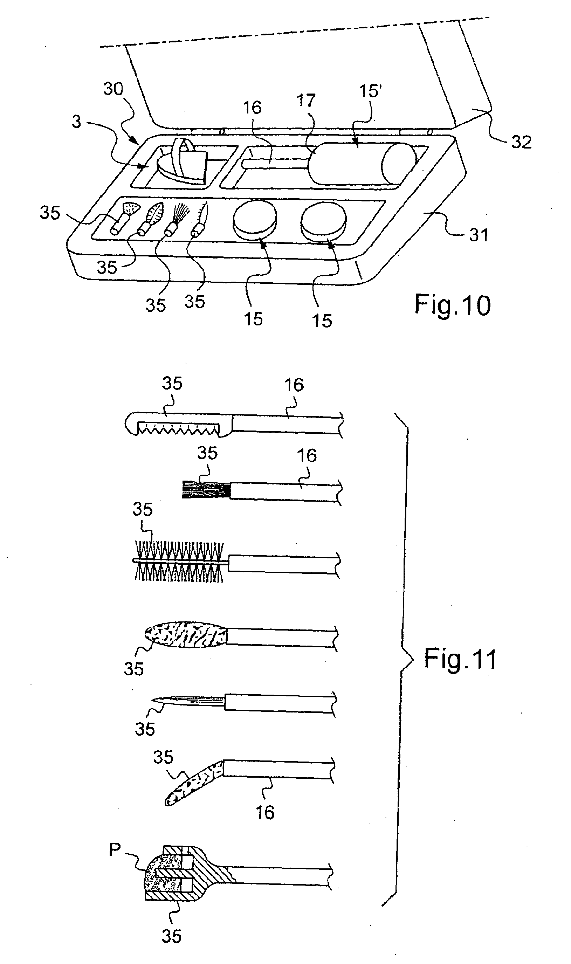

L'ensemble 1 peut être proposé à l'utilisateur dans un système de conditionnement 30 qui peut se présenter sous diverses formes, par exemple sous la forme d'un emballage dans lequel le dispositif 3 et le produit et/ou l'applicateur sont contenus. Il peut s'agir par exemple d'une boîte, d'un blister comme illustré à la figure 9, d'un sachet, d'une trousse ou d'un boîtier, lequel peut comporter par exemple une partie de base 31 et un couvercle 32 articulé sur la partie de base ou pouvant être fixé de façon amovible sur celle-ci, comme illustré à la figure 10.

Le système de conditionnement 30 peut loger outre le dispositif 3 un applicateur 15', qui peut par exemple être prévu pour permettre la fixation de plusieurs éléments d'application 35. Le système de conditionnement 30 peut également comporter un ou plusieurs applicateurs 15 pouvant être fixés sur le doigt directement.

On a illustré à la figure 11, différents exemples d'applicateurs ou d'éléments d'application 35 pouvant être présents dans le système de conditionnement 30 et/ou utilisés avec le dispositif 3, notamment un peigne pour les cils ou les sourcils, un pinceau, une brosse à mascara, un embout en mousse, une pointe feutre, un embout floqué et un applicateur capillaire capable de retenir du produit par capillarité.

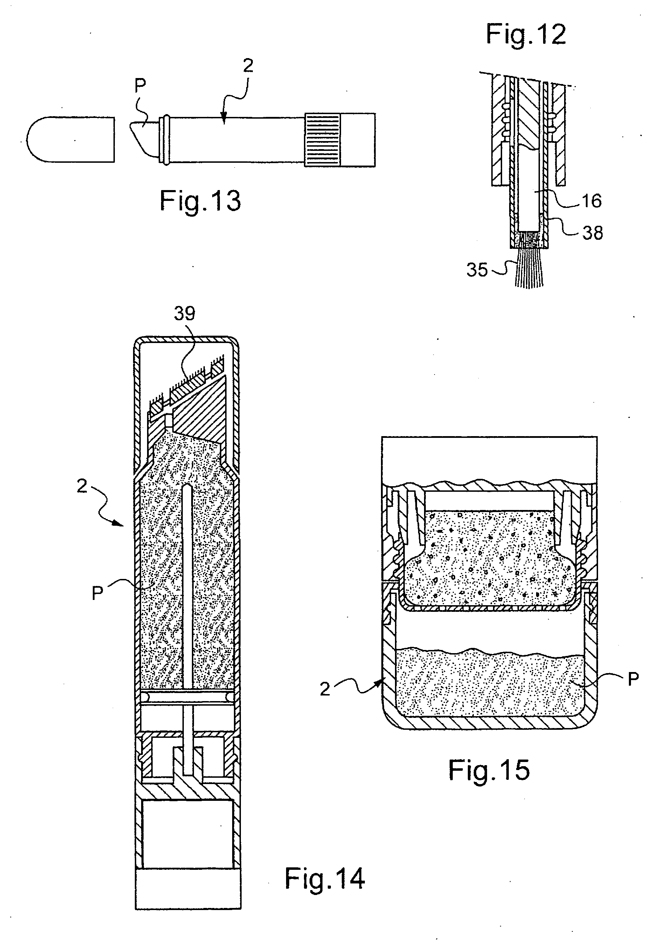

L'applicateur peut également comporter, le cas échéant, comme illustré à la figure 12, une gaine 38 qui peut entourer au moins partiellement l'élément d'application 35 et/ou la tige 16 afin de constituer une réserve de produit.

L'application du produit peut également s'effectuer par exemple au moyen d'un dispositif de conditionnement et d'application de type rouge à lèvres, comme illustré à la figure 13, le produit se présentant par exemple sous la forme d'un stick ou d'un pain de produit ou d'une poudre compactée ou étant contenu sous une forme fluide dans le dispositif et distribué au travers d'un embout 39, comme illustré à la figure 14. L'applicateur peut encore, dans une variante non illustrée, comporter une bille applicatrice de type *roll on* ou un rouleau applicateur.

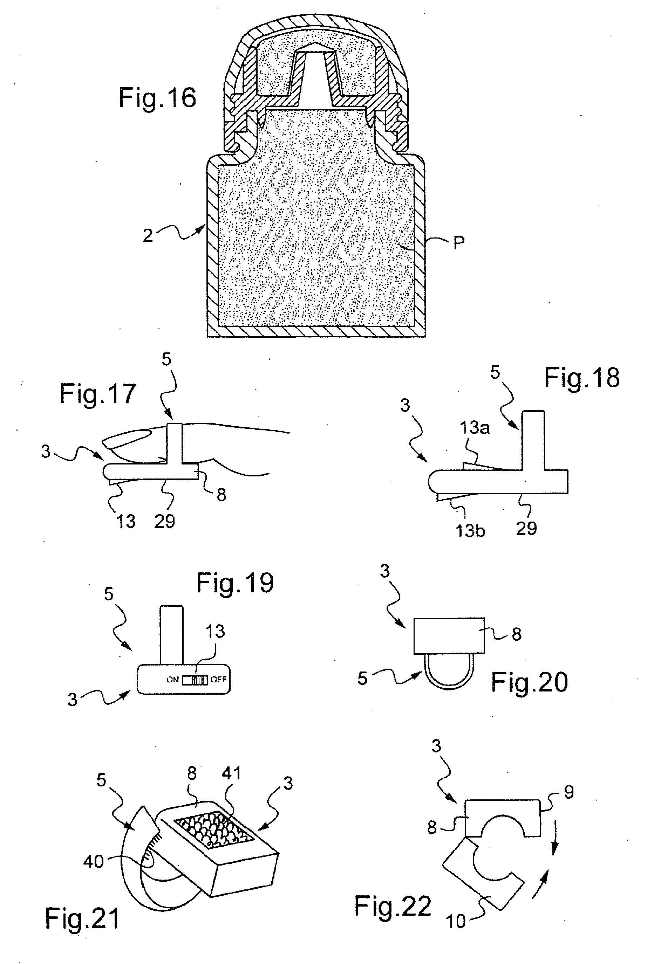

L'applicateur peut encore être un bloc d'un matériau poreux tel que par exemple une mousse ou un fritté. L'applicateur peut être détachable du récipient 2 contenant le produit P, comme illustré à la figure 15 ou rester solidaire du récipient 2 pendant l'application, comme illustré à la figure 16.

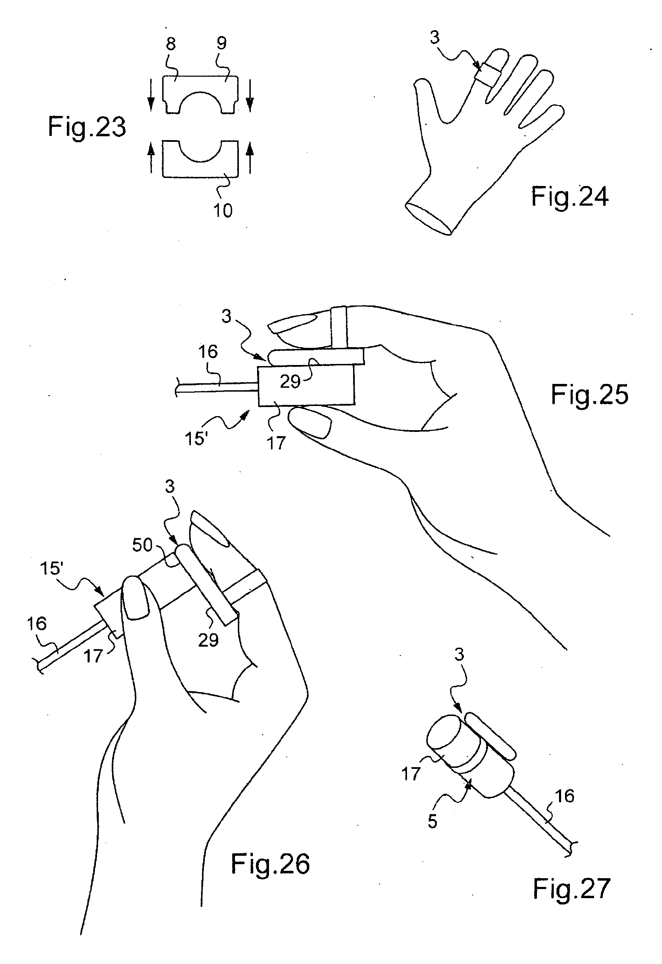

L'organe de commande 13 peut être situé ailleurs que du côté des moyens de fixation 5, par exemple sur la face opposée 29 du boîtier 8, comme illustré à la figure 17. Cela peut permettre par exemple de commander le fonctionnement de la source vibrante seulement lors de sa mise en contact avec l'applicateur 15', comme illustré aux figures 25 et 26.

Le dispositif 3 peut encore comporter, comme illustré à la figure 18, deux organes de commande 13a et 13b respectivement situés du côté des moyens de fixation 5 et du côté opposé 29, afin par exemple de constituer une sécurité supplémentaire, l'enfoncement simultané à la fois de l'organe de commande 13a et de l'organe de commande 13b étant par exemple nécessaire pour commander le fonctionnement de la source vibrante, ce qui peut permettre d'éviter un déclenchement intempestif.

L'organe de commande 13 peut encore se présenter sous la forme d'un interrupteur qui n'est pas actionné automatiquement lors de la mise en place du doigt ni lors du contact avec l'applicateur, comme illustré à la figure 19.

L'organe de commande peut, dans des variantes non illustrées, se présenter sous la forme d'un interrupteur à effleurement, voire sans contact par effet capacitif, ou encore sous la forme d'une commande vocale ou sensible à la température ou à une pression exercée sur les moyens de fixation. Le fonctionnement ou l'arrêt de la source vibrante 4 peut encore être déclenché par la mise en place de la source électrique 6 lorsque celle-ci est logée dans le boîtier 8 du dispositif 3 ou, lorsque la source 6 est logée dans l'applicateur, par l'établissement d'un contact électrique entre l'applicateur et le dispositif 3.

Les moyens de fixation 5 peuvent être réalisés de multiples manières et, comme illustré à la figure 20, peuvent par exemple comporter une portion en forme d'arche refermée par le boîtier 8. En variante, comme illustré à la figure 21, les moyens de fixation 5 peuvent comporter une structure souple qui est par exemple munie d'un premier moyen d'accrochage 40 pouvant coopérer avec un deuxième moyen d'accrochage 41 prévu sur le boîtier 8, par exemple des moyens d'accrochage de type Velcro^{®} à crochets et boucles ou tout autre moyen d'accrochage tel que par exemple une fixation à bouton-pression, à adhésif magnétique, à encliquetage, à glissière, à bracelet, ou à lacet, les moyens de fixation 5 pouvant alors dans ce cas comporter par exemple deux liens chacun solidaires du boîtier et à nouer autour du doigt ou du poignet. Lorsque l'un des moyens d'accrochage est adhésif, celui-ci peut être repositionnable ou non.

Le boîtier 8 du dispositif 3 peut encore comporter deux parties 9 et 10 qui peuvent être mobiles l'une par rapport à l'autre et qui peuvent être refermées autour du doigt, par exemple deux parties articulées comme illustré à la figure 22 ou deux parties à assembler l'une sur l'autre comme illustré à la figure 23, l'assemblage s'effectuant par exemple par encliquetage, par glissière, par fixation magnétique ou autre, par exemple grâce à un élément de verrouillage rapporté. Le dispositif 3 peut encore être maintenu sur le doigt ou la main au moyen d'un gant, lequel peut être complet comme illustré à la figure 24, ou n'être engagé que sur certains doigts seulement, à la façon d'un gant de cycliste.

Dans une variante non illustrée relative à un exemple de mise en oeuvre du procédé de traitement selon l'invention, le dispositif 3 ne comporte pas de moyens de fixation, étant maintenu dans la paume de la main ou entre deux doigts dans la région interdigitale durant l'application ou le prélèvement.

Selon la manière dont le dispositif d'aide est amené au contact de l'applicateur ou du positionnement du dispositif d'aide sur le doigt, on peut par exemple modifier l'orientation des vibrations transmises à l'applicateur.

Cela peut par exemple être utile lors du maquillage en fonction de l'effet recherché, l'orientation des vibrations transmises à l'applicateur étant dans le cas du maquillage de cils préférentiellement orientée parallèlement aux cils pour lisser le produit sur ceux-ci.

Sur la figure 25, on a illustré un contact entre l'applicateur 15' et la face 29 du dispositif 3 opposée au doigt, le long d'une génératrice de l'organe de préhension 17, et dans l'exemple de la figure 26, le contact entre le dispositif 3 et l'organe de préhension 17 a lieu en bout 50 de celui-ci. Sur cette figure, le dispositif 3 peut avantageusement comporter un organe de commande qui est actionné par la mise en contact de l'applicateur 15' avec le dispositif 3, notamment dès qu'une certaine pression est exercée par l'applicateur 15' sur le dispositif 3, ce qui permet de garantir une bonne transmission des vibrations.

Avec un même dispositif 3, on peut faire vibrer un élément d'application de différentes manières en fonction de la zone de l'applicateur où on applique la source vibrante. On peut par exemple balader le doigt muni du dispositif 3 sur l'applicateur pour obtenir des vibrations différentes.

On a illustré à la figure 27 la possibilité de réaliser les moyens de fixation 5 de telle manière à ce qu'ils puissent se fixer directement sur l'applicateur, notamment sur l'organe de préhension 17. Dans une telle réalisation notamment, la source électrique peut être contenue dans l'organe de préhension 17, cette dernière comportant par exemple deux contacts électriques pour transmettre le courant nécessaire à la source vibrante contenue dans le dispositif 3.

Les contacts électriques du dispositif 3 peuvent être présents sur le boîtier ou sur les moyens de fixation 5.

Le cas échéant, un cordon électrique miniature peut être prévu entre l'applicateur et le dispositif 3.

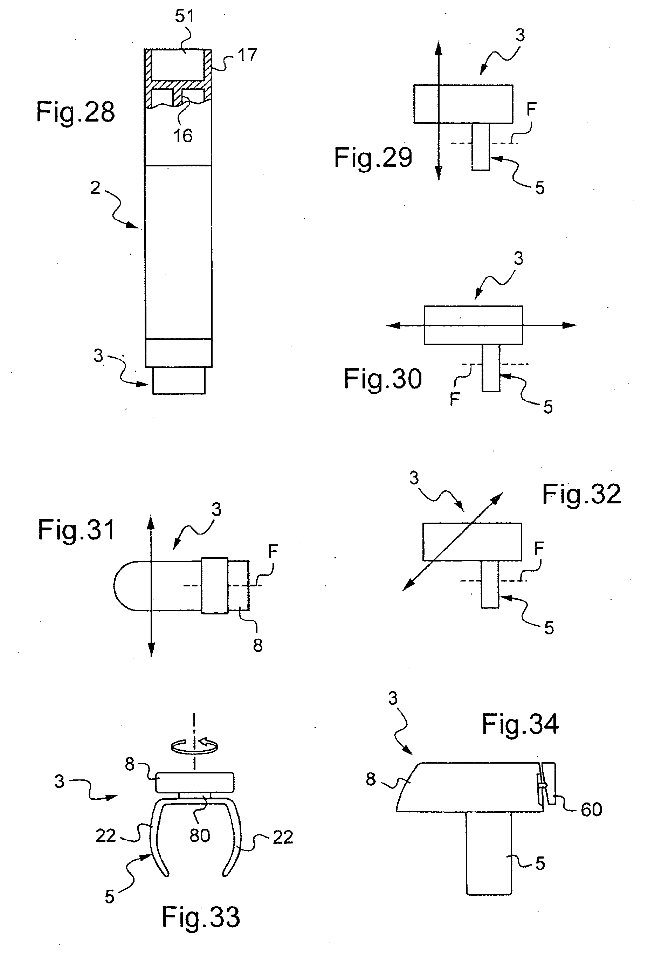

Dans la variante illustrée à la figure 28, le dispositif 3 est agencé pour pouvoir se fixer de façon amovible sur le récipient 2 contenant le produit à appliquer, notamment en l'absence d'utilisation ou lors du prélèvement du produit.

L'applicateur peut être agencé pour se fixer sur le récipient 2 en l'absence d'utilisation, de façon à fermer celui-ci. L'organe de préhension 17 peut comporter un logement 51 permettant de fixer le dispositif 3 sur l'applicateur afin de le solidariser avec l'applicateur. Une telle solidarisation peut améliorer la transmission des vibrations du dispositif 3 vers l'applicateur. Cela peut encore permettre de loger dans l'applicateur la source électrique 6. Dans l'exemple de la figure 28, le dispositif 3 peut comporter des moyens de fixation 5 sur le doigt, non apparents sur cette figure.

Diverses orientations des vibrations de la source vibrante 4 relativement aux moyens de fixation 5 sont possibles, comme illustré aux figures 29 à 32 qui ne représentent pas toutes les possibilités. La source vibrante peut par exemple vibrer transversalement à l'axe F du doigt, comme illustré sur la figure 29, sensiblement parallèlement à l'axe F du doigt comme illustré à la figure 30, sensiblement perpendiculairement à l'axe F du doigt et perpendiculairement également à l'axe longitudinal du boîtier 8, comme illustré à la figure 31, ou encore obliquement relativement à l'axe F du doigt, comme illustré à la figure 32.

L'orientation des vibrations peut être fixe relativement aux moyens de fixation 5 ou réglable, par exemple grâce à l'utilisation d'une articulation 80 entre le boîtier 8 et les moyens de fixation 5, comme illustré à la figure 33, ou grâce à un montage des moyens de fixation sur le boîtier offrant plusieurs possibilités d'orientation.

Dans une variante, le dispositif 3 peut comporter deux sources vibrantes vibrant selon des directions différentes et un moyen de sélection de l'une ou l'autre de ces sources vibrantes en fonction de la direction des vibrations recherchées.

Le dispositif 3 peut également comporter un moyen de réglage de l'amplitude des vibrations, par exemple un moyen électronique tel qu'un potentiomètre rotatif ou à curseur ou une ou plusieurs touches de commande qui permettent par exemple de régler l'amplitude de la tension ou du courant d'alimentation de la source vibrante.

Le dispositif 3 peut encore comporter un moyen mécanique pour agir sur l'amplitude des vibrations, par exemple un curseur 60 qui permet de modifier la position et/ou l'orientation de la source vibrante à l'intérieur du boîtier 8 et/ou d'amener ou non au contact de celle-ci un élément d'amortissement et/ou de modifier la pression exercée par un élément d'amortissement sur la source vibrante et/ou d'agir sur la manière dont la source vibrante est fixée sur le boîtier 8.

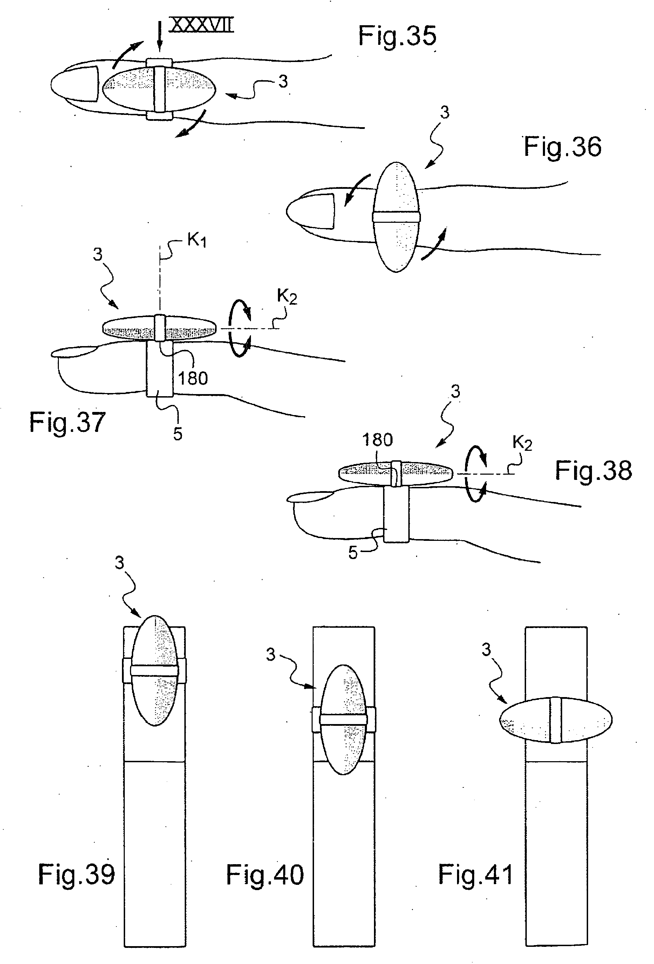

On a représenté aux figures 35 à 38 une autre variante de réalisation du dispositif 3.

Dans cet exemple, le boîtier logeant la source vibrante est monté de façon à pouvoir tourner relativement aux moyens de fixation 5 autour d'un premier axe K₁, grâce à un élément d'articulation 180.

Cela permet d'orienter le boîtier soit avec son axe longitudinal sensiblement parallèle à celui du doigt, comme illustré à la figure 35, soit avec son axe longitudinal sensiblement perpendiculaire à l'axe longitudinal du doigt, comme illustré à la figure 36.

Cela peut permettre de modifier l'orientation des vibrations transmises au doigt.

Dans l'exemple considéré, le boîtier peut également tourner relativement à l'élément d'articulation 180 autour d'un deuxième axe de rotation K₂, ce qui peut permettre de modifier l'orientation de vibrations relativement au doigt, le boîtier pouvant par exemple tourner d'un quart de tour entre les configurations illustrées aux figures 37 et 38.

Dans une variante non illustrée, l'élément 180 est fixe relativement aux moyens de fixation 5 et le boîtier peut seulement tourner autour de l'axe K₂.

Les moyens de fixation 5 peuvent permettre le cas échéant, comme illustré aux figures 39 à 41, le montage du dispositif 3 sur l'applicateur à différents niveaux et/ou avec différentes orientations, en fonction par exemple de l'intensité et/ou de l'orientation des vibrations recherchées.

A la figure 42, on a illustré la possibilité de réaliser à proximité de l'organe de commande 13 au moins un relief antidérapant 200, par exemple de forme annulaire. Un tel relief 200, réalisé par exemple par surmoulage d'une matière élastomère sur le boîtier logeant la source vibrante, peut améliorer la transmission des vibrations entre la source vibrante et l'applicateur auquel les vibrations doivent être transmises. Le relief 200 comporte par exemple une ou plusieurs nervures annulaires.

L'invention n'est pas limitée aux exemples qui viennent d'être décrits. On peut notamment combiner entre elles les particularités de réalisation des différents exemples illustrés au sein de variantes non illustrées. Par exemple, l'un quelconque des moyens de fixation 5 peut équiper l'un quelconque des dispositifs 3.

L'invention n'est pas limitée à un applicateur particulier et ce dernier peut notamment être muni d'un moyen permettant de chauffer le produit et/ou les cils lors de l'application.

Le cas échéant, le dispositif 3 peut être agencé de manière à pouvoir être rechargé en électricité en étant reposé sur un socle ou être alimenté par le secteur par l'intermédiaire d'un transformateur éventuel.

Les applicateurs utilisés pourront être à usage unique, le cas échéant.

Le produit peut présenter toute rhéologie et consistance. Le produit peut par exemple être une pâte, un gel, un liquide ou une poudre, compactée ou libre.

L'application du produit peut se faire, notamment lorsque l'invention est mise en oeuvre pour appliquer un produit sur les fibres kératiniques, après avoir chauffé le produit, par exemple par passage dans un four à micro-ondes.

L'expression « comportant un » doit être comprise comme étant synonyme de « comportant au moins un », sauf si le contraire est spécifié.

## Revendications

1. Ensemble d'application d'un produit cosmétique ou dermatologique, comportant :
- un produit de maquillage et/ou un applicateur (15, 15') configuré pour appliquer le produit sur des matières kératiniques,
- un dispositif (3) d'aide à l'application du produit, dépourvu de produit, ce dispositif comportant des moyens (5) de fixation amovible à au moins un doigt et une source vibrante (4) permettant de produire des vibrations.

2. Ensemble selon la revendication 1, comportant un moyen de conditionnement contenant le produit et/ou l'applicateur et le dispositif d'aide à l'application.

3. Ensemble selon la revendication 1 ou 2, contenant un produit de maquillage.

4. Ensemble selon l'une quelconque des revendications 1 à 3, comportant un applicateur et le produit à appliquer.

5. Ensemble selon la revendication 4, l'applicateur étant agencé pour fermer un récipient contenant le produit.

6. Ensemble selon l'une des revendications précédentes, les moyens de fixation étant agencés pour permettre la fixation sur l'un au moins de l'index, du pouce, du majeur, sur la troisième phalange d'un doigt, la deuxième phalange d'un doigt, la première phalange d'un doigt.

7. Ensemble selon l'une quelconque des revendications 1 à 6, les moyens de fixation comportant au moins une portion annulaire ou semi-annulaire à travers laquelle le doigt peut être introduit.

8. Ensemble selon l'une quelconque des revendications 1 à 6, les moyens de fixation comportant au moins une pince pouvant enserrer le doigt.

9. Ensemble selon l'une quelconque des revendications 1 à 6, les moyens de fixation comportant au moins une structure pouvant être refermée sur le doigt.

10. Ensemble selon l'une quelconque des revendications 1 à 6, les moyens de fixation comportant une portion en forme de doigt de gant, de gant ou de dé.

11. Ensemble selon l'une quelconque des revendications 1 à 6, les moyens de fixation comportant deux portions à assembler sur le doigt.

12. Ensemble selon l'une quelconque des revendications précédentes, comportant un organe de commande (13) du fonctionnement de la source vibrante.

13. Ensemble selon la revendication 12, l'organe de commande étant à déclenchement par enfoncement.

14. Ensemble selon la revendication 13, l'organe de commande étant disposé de telle sorte que l'enfoncement s'effectue à la mise en place du doigt.

15. Ensemble selon la revendication 13, l'organe de commande étant disposé de telle sorte que l'enfoncement s'effectue en réponse à la mise en contact du dispositif avec l'applicateur.

16. Ensemble selon l'une quelconque des revendications précédentes, comportant deux organes (13a, 13b) de commande du fonctionnement de la source vibrante, notamment sur deux faces respectives du dispositif.

17. Ensemble selon l'une quelconque des revendications précédentes, la source vibrante comportant un moteur permettant de produire des vibrations, notamment un moteur entraînant une masselotte ou un excentrique, un vibreur piézoélectrique ou électromécanique.

18. Ensemble selon la revendication 17, la source vibrante comportant un moteur en forme de disque.

19. Ensemble selon l'une quelconque des revendications précédentes, le dispositif (3) comportant une source électrique (6).

20. Ensemble selon l'une quelconque des revendications 1 à 18, le dispositif (3) étant dépourvu de source électrique, celle-ci étant présente dans l'applicateur.

21. Ensemble selon l'une quelconque des revendications précédentes, comportant un organe de réglage permettant à l'utilisateur de régler la fréquence et/ou l'amplitude des vibrations.

22. Ensemble selon l'une quelconque des revendications précédentes, le dispositif permettant de régler l'orientation des vibrations par rapport aux moyens de fixation.

23. Ensemble selon l'une quelconque des revendications précédentes, comportant au moins deux applicateurs différents et/ou deux produits différents.

24. Ensemble selon l'une quelconque des revendications précédentes, les moyens de fixation étant agencés pour permettre la fixation du dispositif sur l'applicateur et/ou sur un récipient contenant le produit.

25. Ensemble selon l'une quelconque des revendications 1 à 24, la fréquence des vibrations de la source vibrante étant comprise entre 1 et 500 Hz, mieux 10 et 300 Hz, encore mieux 50 et 200 Hz.

26. Ensemble selon la revendication 12, le dispositif (3) comportant un relief antidérapant (200) à proximité de l'organe de commande (13).

27. Ensemble selon l'une quelconque des revendications 1 à 26, le produit étant à usage externe.

28. Procédé de traitement cosmétique, dans lequel on applique un produit cosmétique sur des matières kératiniques après avoir fixé sur le doigt un dispositif (3) d'aide à l'application selon l'une quelconque des revendications 1 à 26.

29. Procédé de maquillage des matières kératiniques, dans lequel on applique un produit de maquillage sur les matières kératiniques après avoir fixé sur le doigt un dispositif d'aide à l'application l'une quelconque des revendications 1 à 26 ou en tenant dans la main ou entre les doigts, lors de l'application, un tel dispositif.

30. Procédé selon l'une des deux revendications précédentes, dans lequel le dispositif (3) d'aide à l'application est fixé sur le doigt.

31. Procédé selon l'une quelconque des revendications 28 à 30, dans lequel le produit est appliqué au moyen du doigt en se servant de l'extrémité du doigt comme surface d'application.

32. Procédé selon l'une quelconque des revendications 28 à 31, dans lequel le produit est appliqué au moyen d'un applicateur (15, 15').

33. Procédé selon la revendication précédente, dans lequel le dispositif d'aide à l'application est sans contact avec l'applicateur lors de l'application du produit.

34. Procédé selon l'une quelconque des revendications 28 à 32, dans lequel on amène le dispositif (3) d'aide à l'application au contact de l'applicateur pour lui transmettre des vibrations lors de l'application.

35. Procédé selon l'une quelconque des revendications 28 à 34, dans lequel les vibrations sont exercées durant l'application du produit sur les matières kératiniques.

36. Procédé selon l'une quelconque des revendications 28 à 35, dans lequel les vibrations sont exercées durant le prélèvement du produit.

37. Procédé selon l'une quelconque des revendications 28 à 36, dans lequel les matières kératiniques sont constituées par la peau.

38. Procédé selon l'une quelconque des revendications 28 à 36, dans lequel les matières kératiniques sont constituées par les lèvres.

39. Procédé selon l'une quelconque des revendications 28 à 36, dans lequel les matières kératiniques sont constituées par des fibres kératiniques notamment les cils, sourcils, ou cheveux.

40. Procédé selon l'une quelconque des revendications 28 à 36, les matières kératiniques étant constituées par les ongles.

41. Procédé selon la revendication 32, dans lequel le contact du dispositif d'aide avec l'applicateur s'effectue par une face de l'applicateur orientée sensiblement perpendiculairement à un axe longitudinal de l'applicateur.

42. Procédé selon la revendication 32, dans lequel le contact entre l'applicateur et le dispositif d'aide s'effectue par une face de l'applicateur orientée sensiblement parallèlement à un axe longitudinal de l'applicateur.

## Claims

1. Assembly for applying a cosmetic or dermatological product, comprising:
a make-up product and/or an applicator (15, 15') configured for applying the product to keratinous materials; and
- a device (3), devoid of product, for assisting in the application of the product, this device comprising means (5) for removably fastening it to at least one finger and a vibrating source (4) for producing vibrations.

2. Assembly according to Claim 1, which includes a packaging means containing the product and/or the applicator and the application assistance device.

3. Assembly according to Claim 1 or 2, which contains a make-up product.

4. Assembly according to any one of Claims 1 to 3, which comprises an applicator and the product to be applied.

5. Assembly according to Claim 4, the applicator being designed to close a container that contains the product.

6. Assembly according to one of the preceding claims, the fastening means being designed to be fastened onto at least the index finger, the thumb or the middle finger, on the third phalange of a finger, the second phalange of a finger or the first phalange of a finger.

7. Assembly according to any one of Claims 1 to 6, the fastening means having at least one annular or semi-annular portion through which the finger may be introduced.

8. Assembly according to any one of Claims 1 to 6, the fastening means comprising at least one clip that can grip the finger.

9. Assembly according to any one of Claims 1 6, the fastening means having at least one structure that can be closed around the finger.

10. Assembly according to any one of Claims 1 to 6, the fastening means having a portion in the form of a fingerstall, glove or thimble.

11. Assembly according to any one of Claims 1 to 6, the fastening means having two portions to be assembled on the finger.

12. Assembly according to any one of the preceding claims, which includes a control member (13) for operating the vibrating source.

13. Assembly according to Claim 12, the control member being one triggered by a depressing action.

14. Assembly according to Claim 13, the control member being placed in such a way that the depressing action is performed on placing the device on the finger.

15. Assembly according to Claim 13, the control member being placed in such a way that the depressing action is performed in response to the device being brought into contact with the applicator.

16. Assembly according to any one of the preceding claims, which has two control members (13a, 13b) for operating the vibrating source, especially on two respective faces of the device.

17. Assembly according to any one of the preceding claims, the vibrating source comprising a motor for producing vibrations, especially a motor driving a flyweight or an eccentric, a piezoelectric vibrator or an electromechanical vibrator.

18. Assembly according to Claim 17, the vibrating source comprising a disc-shaped motor.

19. Assembly according to any one of the preceding claims, the device (3) including an electrical source (6).

20. Assembly according to any one of Claims 1 to 18, the device (3) containing no electrical source, the latter being present in the applicator.

21. Assembly according to any one of the preceding claims, which includes an adjustment member allowing the user to adjust the frequency and/or the amplitude of the vibrations.

22. Assembly according to any one of the preceding claims, the device allowing the orientation of the vibrations relative to the fastening means to be adjusted.

23. Assembly according to any one of the preceding claims, which comprises at least two different applicators and/or two different products.

24. Assembly according to any one of the preceding claims, the fastening means being designed to allow the device to be fastened to the applicator and/or to a container containing the product.

25. Assembly according to any one of Claims 1 to 24, the frequency of the vibrations of the vibrating source being between 1 and 500 Hz, better still between 10 and 300 Hz and even better between 50 and 200 Hz.

26. Assembly according to Claim 12, the device (3) having a non-slip feature (200) close to the control member (13).

27. Assembly according to any one of Claims 1 to 26, the product being for external usage.

28. Cosmetic treatment method in which a cosmetic product is applied to keratinous materials after an application assistance device (3) according to any one of Claims 1 to 26 has been fastened onto a finger.

29. Method of applying make-up to keratinous materials, in which a make-up product is applied to the keratinous materials after an application assistance device according to any one of Claims 1 to 26 has been fastened onto a finger or in which such a device is held in one's hand or between two fingers during application.

30. Method according to either of the preceding two claims, in which the application assistance device (3) is fastened to a finger.

31. Method according to any one of Claims 28 to 30, in which the product is applied by means of a finger using the tip of the finger as application surface.

32. Method according to any one of Claims 28 to 31, in which the product is applied by means of an applicator (15, 15').

33. Method according to the preceding claim, in which the application assistance device is not in contact with the applicator during application of the product.

34. Method according to any one of Claims 28 to 32, in which the application assistance device (3) is brought into contact with the applicator in order to transmit vibrations to it during application.

35. Method according to any one of Claims 28 to 34, in which the vibrations are exerted during application of the product to the keratinous materials.

36. Method according to any one of Claims 28 to 35, in which the vibrations are exerted during pick-up of the product.

37. Method according to any one of Claims 28 to 36, in which the keratinous materials consist of the skin.

38. Method according to any one of Claims 28 to 36, in which the keratinous materials consist of the lips.

39. Method according to any one of Claims 28 to 36, in which the keratinous materials consist of keratinous fibres, especially eyelashes, eyebrows or hair.

40. Method according to any one of Claims 28 to 36, the keratinous materials consisting of the nails.

41. Method according to Claim 32, in which the assistance device comes into contact with the applicator via an applicator face oriented substantially perpendicular to a longitudinal axis of the applicator.

42. Method according to Claim 32, in which the applicator comes into contact with the assistance device via an applicator face oriented substantially parallel to a longitudinal axis of the applicator.

## Patentansprüche

1. Anordnung zur Applikation eines kosmetischen oder dermatologischen Produktes, die aufweist:
- ein Produkt zum Schminken und/oder eine Applikationseinrichtung (15, 15'), die konstruiert ist, um das Produkt auf keratinischen Materialien aufzutragen,
- eine Vorrichtung (3) zur Unterstützung der Applikation des Produktes, entblößt von Produkt, weist diese Vorrichtung Mittel (5) zur abnehmbaren Festlegung von zumindest einem Finger und einer Vibrationsquelle (4) auf, die es ermöglicht, Vibrationen zu erzeugen.

2. Anordnung nach anspruch 1, die ein Mittel zur Aufbereitung aufweist, das das Produkt und/oder die Applikationsvorrichtung und die Vorrichtung zur Unterstützung der Applikation enthält.

3. Anordnung nach dem Anspruch 1 oder 2, die ein Schminkprodukt enthält.

4. Anordnung nach irgendeinem der Ansprüche 1 bis 3, die eine Applikationseinrichtung und das zu applizierende Produkt aufweist.

5. Anordnung nach anspruch 4, wobei die Applikationseinrichtung ausgebildet ist, um einem Behälter, der das Produkt enthält, zu verschließen.

6. Anordnung nach irgendeinem der voranstehenden Ansprüche, wobei die Mittel zur Festlegung angeordnet sind, um die Festlegung auf zu mindestens einem von dem Zeigefinger, dem Daumen, der Mittelfinger, auf dem dritten Fingerglied eines Fingers, dem zweiten Fingerglied von einem Finger, dem ersten Fingerglied von einem Finger zu erlauben.

7. Anordnung nach irgendeinem der Ansprüche 1 bis 6, wobei die Mittel zur Festlegung zumindest einen ringförmigen oder halbringförmigen Abschnitt aufweisen, durch welchen der Finger quer eingeführt werden kann.

8. Anordnung nach irgendeinem der Ansprüche bis 6, wobei die Mittel zur Festlegung zumindest einen Griff bzw. eine Klemme aufweisen, die den Finger umschlingen kann.

9. Anordnung nach irgendeinem der Ansprüche bis 6, wobei die Mittel zum Festlegen zumindest eine Konstruktion aufweisen, die auf dem Finger sperrbar sein kann.

10. Anordnung nach irgendeinem der Ansprüche 1 bis 6, wobei die Mittel zum Festlegen einen Abschnitt in der Form eines Fingerhandschuhs, eines Handschuhs oder eines Fingerhuts ausweisen.

11. Anordnung nach irgendeinem der Ansprüche 1 bis 6, wobei die Mittel zum Festlegen zwei Abschnitte zum Zusammenbau bzw. Zusammenfügen auf dem Finger aufweisen.

12. Anordnung nach irgendeinem der voranstehenden Ansprüche, die ein Betätigungselement (13) für die Funktion der Vibrationsquelle aufweist.

13. Anordnung nach Anspruch 12, wobei das Betätigungselement zum Auslösen durch Ein- bzw. Niederdrücken ist,

14. Anordnung nach Anspruch 13, wobei das Betätigungselement in der Weise angeordnet ist, dass die Auslösung durch das Anlegen eines Fingers bewirkt wird.

15. Anordnung nach Anspruch 13, wobei das Betätigungs- bzw. Bedienelement in der Weise angeordnet ist, um das Ein- bzw. Niederdrücken in Reaktion auf das Inkontaktbringen der Vorrichtung mit der Applikationseinrichtung bewirkt wird.

16. Anordnung nach irgendeinem der voranstehenden Ansprüche, die zwei Elemente (13a, 13b) zum Bedienen bzw. Betätigen der Funktion der Vibrationsquelle ausweist, insbesondere auf zwei jeweiligen Flächen der Vorrichtung.

17. Anordnung nach irgendeinem der voranstehenden Ansprüche, wobei die Vibrationsquelle einen Motor aufweist, der es ermöglicht, Vibrationen zu erzeugen, insbesondere einen Massekopf bzw. Fliehgewicht, oder ein Exzenter, einen piezoelektrischen oder elektromechanischen Vibrator.

18. Anordnung nach Ansprach 17, wobei die Vibrationsquelle einen Motor mit der Form einer Scheibe aufweist.

19. Anordnung nach irgendeinem der voranstehenden Ansprüche, wobei die Vorrichtung (3) eine elektrische Quelle (6) aufweist.

20. Anordnung nach irgendeinem der Ansprüche 1 bis 18, wobei die Vorrichtung (3) ohne elektrische Quelle ausgebildet ist, wobei diese in der Applikationseinrichtung zugegen ist.

21. anordnung nach irgendeinem der voranstehenden Ansprüche, die ein Regelelement aufweist, das es eine Verwender ermöglicht, die Frequenz und/oder die Amplitude der Vibrationen zu regeln.

22. Anordnung nach irgendeinem der voranstehenden Ansprüche, wobei es die Vorrichtung ermöglicht, die Orientierung der Vibrationen in Bezug auf die Mittel zum Festlegen zu regeln.

23. Anordnung nach irgendeinem der voranstehenden Ansprüche, die zumindest zwei verschiedene Applikationseinrichtungen und/oder zwei verschiedene Produkte aufweist,

24. Anordnung nach irgendeinem der voranstehenden Ansprüche, wobei die Mittel zum Befestigen angeordnet sind, um die Befestigung der Vorrichtung auf der Applikationseinrichtung und/oder auf einem Behälter, der das Produkt enthält, zu ermöglichen.

25. Anordnung nach irgendeinem der Ansprüche 1 bis 24, wobei die Frequenz der Vibrationen der Vibrationsquelle zwischen 1 und 500 Hz, besser 10 und 300 Hz, noch besser 50 bis 200 Hz vorkommt.

26. Anordnung nach anspruch 12, wobei die Vorrichtung (3) eine Antirutschstruktur (200) in der Nähe des Bedienelementes (13) aufweist.

27. Anordnung nach den voranstehenden Ansprüchen 1 bis 26, wobei das Produkt zur externen bzw, äußerlichen Verwendung ist.

28. Verfahren zur kosmetischen Behandlung, bei welchem man ein kosmetisches Produkt auf einem keratinischen Material anwendet, nachdem auf den Fingern eine Vorrichtung (3) zur Unterstützung der Applikation festgelegt worden ist, nach irgendeinem der Ansprüche 1 bis 26.

29. Verfahren zum Schminken von keratinischen Materialien, bei welchem man ein Produkt zum Schminken auf die keratinische Materialien appliziert, nachdem auf dem Finger eine Vorrichtung zur Unterstützung bei der Applikation festgelegt worden ist, nach irgendeinem der Ansprüche 1 bis 26, oder indem eine solche Vorrichtung während des Applizierens in die Hand oder zwischen die Finger genommen wird.

30. Verfahren nach irgendeinem der voranstehenden Ansprüche, bei welchem die Vorrichtung (3) zum Unterstützen bei der Applikation auf bzw. an dem Finger festgelegt wirt.

31. Verfahren nach irgendeinem der Ansprüche 28 bis 30, bei welchem das Produkt mittels eines Fingers appliziert wird, indem das Ende des Fingers als eine Applikationsoberfläche dient.

32. Verfahren nach irgendeinem der Ansprüche 28 bis 3 1, bei welchem das Produkt mittels einer Applikationseinrichtung (15, 15') appliziert wird.

33. Verfahren nach dem voranstehenden Anspruch, bei welchem die Vorrichtung zur Unterstützung bei der Applikation ohne Berührung mit der Applikationseinrichtung bei der Applikation des Produktes ist.

34. Verfahren nach irgendeinem der Ansprüche 28 bis 32, bei welchem man die Vorrich-(3) zur Unterstützung bei der Applikation in Berührung zu der Applikationseinrichtung hält, um darauf Vibrationen während der Applikation zu übertragen.

35. Verfahren nach irgendeinem der Ansprüche 28 bis 34, bei welchem die Vibrationen. über die Dauer der Applikation des Produktes auf die keratinischen Materialien ausgeübt werden.

36. Verfahren nach irgendeinem der Ansprüche 28 bis 35, bei welchem die Vibrationen während der Entnahme des Produktes ausgeübt werden.

37. Verfahren nach irgendeinem der Ansprüche 28 bis 36, bei welchem die keratinischen Materialien durch die Haut gebildet werden.

38. Verfahren nach irgendeinem der Ansprüche 28 bis 36, bei welchem die keratinischen Materialien durch die Lippen ausgebildet werden.

39. Verfahren nach irgendeinem der Ansprüche 28 bis 36, bei welchem die keratinischen Materialien durch keratinische Fasern gebildet sind, insbesondere die Wimpern, Augenbrauen oder Haare.

40. Verfahren nach irgendeinem der Ansprüche 28 bis 36, wobei die keratinischen Materialien durch Fingernägel gebildet sind.

41. Verfahren nach anspruch 32, bei welchem der Kontakt der Vorrichtung zur Unterstützung mit der Applikationseinrichtung durch eine Fläche der Applikationseinrichtung, die genau senkrecht zu einer Längsachse der Applikationseinrichtung ausgerichtet ist, bewirkt wird.

42. Verfahren nach Anspruch 32, bei welchem der Kontakt zwischen der Applikationseinrichtung und der Vorrichtung zur Unterstützung durch eine Fläche der Applikationseirtrichtung, die genau parallel zu einer Längsachse der Applikationseinrichtung ausgerichtet ist, bewirkt wird.
